# FASCICULE DE BREVET EUROPEEN

(11) **EP 1 076 086 B1**
(45) Date de publication et mention de la délivrance du brevet: **10.08.2005**
(21) Numéro de dépôt: 00402140.8
(22) Date de dépôt: 26.07.2000
(51) Int. Cl.: C10L 1/18, C10L 1/14, C07D 307/12

(54) **Utilisation de dérivés de tétrahydrofuranne comme compositions détergentes pour carburants de type essence.**
Verwendung von Tetrahydrofuran Derivaten als Detergenszusammensetzungen für Benzinkraftstoffe
Use of tetrahydrofurane derivatives as detergent compositions for gasoline fuel

(30) Priorité: 12.08.1999 FR 9910505
(43) Date de publication de la demande: 14.02.2001
(73) Titulaire: Institut Français du Pétrole, 92852 Rueil-Malmaison Cedex (FR)
(72) Inventeur: Delford, Bruno, 75005 Paris (FR); Joly, Stéphane, 78380 Bougival (FR); Lacome, Thierry, 92380 Garches (FR); Gateau, Patrick, 78310 Maurepas (FR); Paille, Fabrice, 78520 Limay (FR)

(56) Documents cités:
- EP-A- 0 064 236
- EP-A- 0 665 206
- WO-A-94/14925
- WO-A-95/17484
- WO-A-98/44022
- DE-A- 3 244 077
- DATABASE CHEMICAL ABSTRACT 18 octobre 1991 (1991-10-18), KAO CORP.: "Furan derivative compositions for defluxing printed circuit boards" XP115:161651

## Description

L'invention concerne l'utilisation de dérivés de tétrahydrofurane comme additifs détergents pour carburants.

On sait que les moteurs d'automobiles ont tendance à former des dépôts à la surface des éléments du moteur en particulier sur les orifices du carburateur, le corps des papillons, les injecteurs de carburant, les orifices et les soupapes d'admission, en raison de l'oxydation et de la polymérisation de divers constituants hydrocarbonés du carburant. Ces dépôts, même lorsqu'ils ne sont présents qu'en faibles quantités, sont souvent responsables de problèmes de conduite importants, tels que le calage du moteur et des défaillances à l'accélération. De plus, les dépôts dans le moteur peuvent accroître de manière importante la consommation de carburant et la production d'émissions polluantes. C'est pourquoi, le développement d'additifs détergents efficaces pour réguler ces dépôts revêt une importance considérable et a déjà fait l'objet de nombreuses recherches.

WO-A-95484 décrit l'utilisation d'éthers aromatiques de poly(oxyalkylène) comme additifs pour carburants.

On a maintenant découvert une nouvelle famille de composés qui présentent une bonne efficacité comme additifs destinés à réduire les dépôts sur les injecteurs et sur les soupapes d'admission.

Les composés utilisables selon l'invention peuvent être définis par la formule générale suivante (I) : 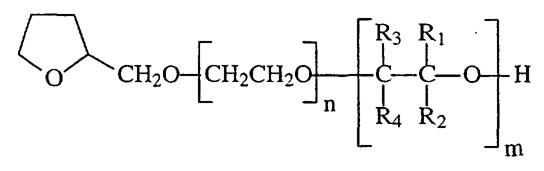 dans laquelle n est un nombre de 0 à 20, de préférence de 0 à 10, R1, R2, R3 et R4 représentent chacun un atome d'hydrogène ou un radical hydrocarboné, par exemple alkyle, de 1 à 30 atomes de carbone, l'un au moins des R1, R2, R3 et R4 étant un radical hydrocarboné, les enchaînements : 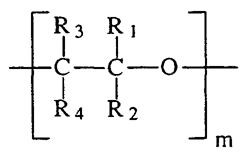 pouvant être constitués de motifs : 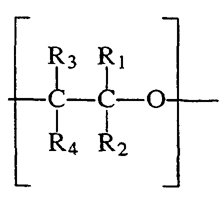 qui diffèrent entre eux par la nature de R1, R2, R3 et/ou R4, et m est un nombre de 1 à 30.

Les composés utilisables selon l'invention se présentent en général sous la forme de mélanges de composés qui diffèrent les uns des autres par la valeur de n et/ou par la nature d'au moins un des radicaux R1 à R4. Il s'agit alors de compositions.

La synthèse des composés ou des compositions défini(e)s ci-dessus peut être effectuée comme décrit ci-après. Elle comprend en général la réaction d'un dérivé hydroxyméthylé du tétrahydrofuranne avec de l'oxyde d'éthylène, puis avec un ou plusieurs autres composés époxydés.

Le dérivé hydroxyméthylé du tétrahydrofuranne, tel que par exemple le 2-(hydroxyméthyl) tétrahydrofuranne est mélangé par exemple à de l'hydrure de sodium. Après avoir purgé, sous agitation, le réacteur de l'hydrogène dégagé, on introduit éventuellement de l'oxyde d'éthylène en une quantité calculée pour obtenir la valeur désirée de n et on porte le milieu à une température de 60 à 130 °C, puis on ajoute l'époxyde ou un mélange d'époxydes de formule générale 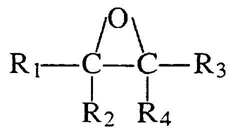 dans laquelle R1, R2, R3 et R4 sont définis comme ci-dessus en une quantité calculée pour obtenir la valeur désirée de m, et on porte le milieu à une température de 80 à 180°C et on maintient le mélange réactionnel à cette température jusqu'à la fin de la consommation du ou des époxydes.

Après retour à la température ambiante, on dilue le milieu avec un solvant organique, par exemple hydrocarboné, tel que l'heptane, on le lave par exemple à l'eau une ou plusieurs fois, puis, après évaporation sous pression réduite de la phase organique, on obtient le produit qui répond à la formule générale (I) :

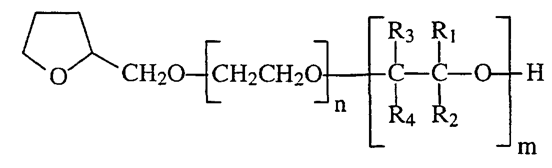

Les compositions de l'invention sont utilisables comme additifs détergents dans les carburants de type essence. Dans cette application, elles peuvent être ajoutées aux carburants à des concentrations par exemple de 20 à 5000 mg par litre. Elles peuvent être aussi utilisées en mélange avec tout autre composé détergent.

Les exemples suivants illustrent l'invention sans en limiter la portée.

### Exemple 1

Dans un réacteur permettant d'opérer sous pression, équipé d'un système d'agitation, d'un système d'introduction de réactifs, d'un système de mesure de la température et de la pression, on introduit 10,2 g (0,1 mole) de 2-tétrahydrofuranyl-hydroxyméthane et 0,12 g d'hydrure de sodium. Après avoir purgé, sous agitation, le réacteur de l'hydrogène dégagé, on introduit 17,6 g (0,4 moles) d'oxyde d'éthylène, puis on porte le milieu à la température de 110 °C et on le maintient à cette température jusqu'à ce que la chute de pression indique la fin de la consommation d'oxyde d'éthylène. Après retour à la température ambiante on introduit 100 g (1,39 mole) de 1,2-époxybutane, puis on porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 50 g d'eau, puis, après évaporation sous pression réduite de la phase organique, on obtient 123 g d'un liquide limpide jaune pâle, soluble dans l'essence et dont la structure correspond à la formule générale : 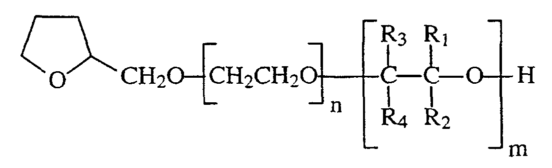 dans laquelle
R1 = R3 = R4 = H ; R2 = -CH2-CH3 ;
n _{moyen} = 4 ; et m _{moyen} = 14.

### Exemple 2

Dans un réacteur permettant d'opérer sous pression, équipé d'un système d'agitation, d'un système d'introduction de réactifs, d'un système de mesure de la température et de la pression, on introduit 10,2g (0,1 mole) de 2-tétrahydrofuranyl-hydroxyméthane et 0,12 g d'hydrure de sodium. Après avoir purgé, sous agitation, le réacteur de l'hydrogène dégagé, on introduit 100 g (1,39 moles) de 1,2-époxybutane. On porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 50 g d'eau, puis, après évaporation sous pression réduite de la phase organique, on obtient 107 g d'un liquide limpide jaune pâle, soluble dans l'essence et dont la structure correspond à la formule générale : 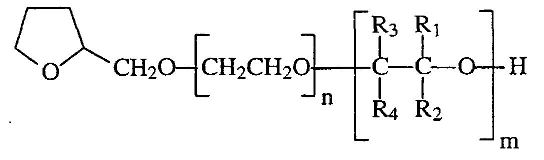 dans laquelle
R1 = R3 = R4 = H ; R2 = -CH2-CH3 ;
n _{moyen} = 0 ; et m _{moyen} = 14.

### Exemple 3

Dans un réacteur permettant d'opérer sous pression, équipé d'un système d'agitation, d'un système d'introduction de réactifs, d'un système de mesure de la température et de la pression, on introduit 10,2g (0,1 mole) de 2-tétrahydrofuranyl-hydroxyméthane et 0,2 g d'hydrure de sodium. Après avoir purgé, sous agitation, le réacteur de l'hydrogène dégagé, on introduit 35,2 g (0,8 mole) d'oxyde d'éthylène, puis on porte le milieu à la température de 110 °C et on le maintient à cette température jusqu'à ce que la chute de pression indique la fin de la consommation d'oxyde d'éthylène. Après retour à la température ambiante, on introduit 130 g (1,81 mole) de 1,2-époxybutane, puis on porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 50 g d'eau, puis après évaporation sous pression réduite de la phase organique, on obtient 170 g d'un liquide limpide jaune pâle, soluble dans l'essence et dont la structure correspond à la formule générale : 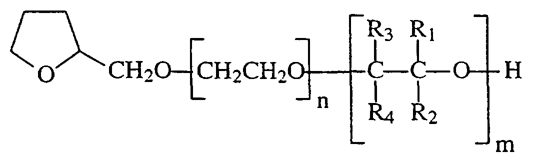 dans laquelle
R1 = R3 = R4 = H ; R = -CH2-CH3 ;
n _{moyen} = 8 ;et m _{moyen} = 14

### Exemple 4

Dans un réacteur permettant d'opérer sous pression, équipé d'un système d'agitation, d'un système d'introduction de réactifs, d'un système de mesure de la température et de la pression, on introduit 102g (0,1 mole) de 2-tétrahydrofuranyl-hydroxyméthane et 0,12 g d'hydrure de sodium. Après avoir purgé, sous agitation, le réacteur de l'hydrogène dégagé, on introduit 17,6g (0,4 mole) d'oxyde d'éthylène, puis on porte le milieu à la température de 110 °C et on le maintient à cette température jusqu'à ce que la chute de pression indique la fin de la consommation d'oxyde d'éthylène. Après retour à la température ambiante, on introduit 129,6 g (1,8 mole) de 1,2-époxybutane, puis on porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 50 g d'eau, puis après évaporation sous pression réduite de la phase organique, on obtient 140 g d'un liquide limpide jaune pâle, soluble dans l'essence et dont la structure correspond à la formule générale : 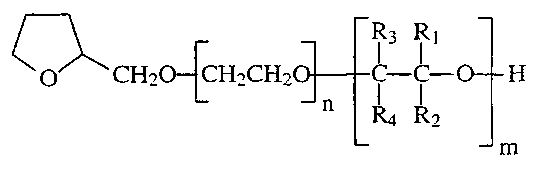 dans laquelle
R1 = R3 = R4 = H ; R = -CH2-CH3 ;
n _{moyen} = 4 ; et m _{moyen} = 18.

### Exemple 5

Les produits préparés dans les exemples précédents sont évalués comme additifs pour leurs propriétés détergentes à une concentration de 400 mg/litre dans une essence sans plomb d'indice d'octane "Recherche" de 96,8 et dont les caractéristiques sont les suivantes :

| | |
|---|---|
| Masse volumique à 15 °C | 753,9 kg/m3 |
| Pression de Vapeur Reid | 60,2 kPa |
| Teneur en plomb | < 2 mg/l |
| Distillation | |
| Point initial | 33,0 °C |
| 5 % | 45,9 °C |
| 10 % | 51,2 °C |
| 20 % | 61,1 °C |
| 30 % | 73,1 °C |
| 40 % | 88,3 °C |
| 50 % | 104,2 °C |
| 60 % | 116,0 °C |
| 70 % | 125,6 °C |
| 80 % | 138,5 °C |
| 90 % | 155,6 °C |
| 95 % | 169,8 °C |
| Point final | 189,0 °C |

L'évaluation est effectuée au moyen d'un essai sur moteur Mercedes M102E selon la méthode CEC-F-05-A-93. La durée de cet essai est de 60 heures. Cette méthode permet d'évaluer la quantité de dépôts formés sur les soupapes d'admission du moteur.

Les résultats qui figurent dans le tableau suivant montrent l'effet des produits de l'invention pour réduire les dépôts sur les soupapes d'admission.

**TABLEAU**

| Additif | Teneur (mg/l) | Masse de dépôt sur les soupapes d'admission (mg) | | | | |
|---|---|---|---|---|---|---|
| | | Soupape 1 | Soupape 2 | Soupape 3 | Soupape 4 | Moyenne |
| Néant | 0 | 241 | 275 | 272 | 312 | 275 |
| Produit de l'Exemple 1 | 400 | 113 | 100 | 108 | 69 | 97,5 |
| Produit de l'Exemple 4 | 400 | 22 | 21 | 29 | 35 | 26,7 |

## Revendications

1. Utilisation comme additif détergent dans une essence d'un composé répondant à la formule générale (I) : 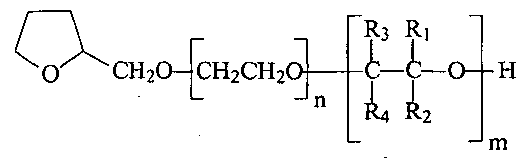 dans laquelle n est un nombre de 0 à 20, R1, R2, R3 et R4 représentent un atome d'hydrogène ou un radical hydrocarboné par exemple alkyle, de 1 à 30 atomes de carbone, l'un au moins des R1, R2, R3 et R4 étant un radical hydrocarboné, et m est un nombre de 1 à 30.

2. Utilisation selon la revendication 1 **caractérisée en ce que**, dans la formule générale (I), les enchaînements 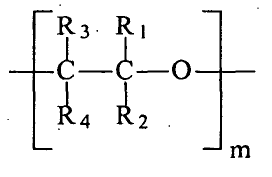 sont constitués de motifs 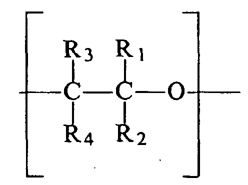 qui diffèrent entre eux par la nature de R1, R2, R3 et/ou R4.

3. Utilisation selon la revendication 1 ou 2 **caractérisée en ce que**, dans la formule (I), les radicaux hydrocarbonés R1, R2, R3 et R4 sont des radicaux alkyles de 1 à 30 atomes de carbone

4. Utilisation selon l'une des revendications 1 à 3 **caractérisée en ce que** dans la formule (I) n est un nombre de 0 à 10 et m est un nombre de 1 à 30.

5. Utilisation comme additif détergent dans une essence d'une composition consistant en un mélange de plusieurs composés tels que définis dans l'une des revendications 1 à 4 qui diffèrent les uns des autres par la valeur de n et/ou par la nature d'au moins un des radicaux R1 à R4.

## Claims

1. Use of a compound of formula I as detergent additive in a gasoline in which n is a number from 0 to 20, R 1, R 2, R 3, and R 4 each represent a hydrogen atom or a hydrocarbon radical for example alkyl, with 1 to 30 carbon atoms, whereby at least one of R 1, R 2, R 3 and R 4 is a hydrocarbon radical and m is a number from 1 to 30.

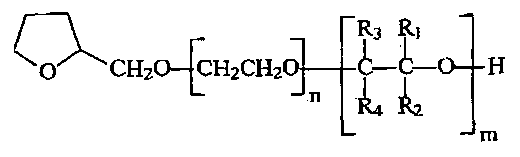

2. Use according to claim 1 **characterized in that**, in formula I, The concatenations 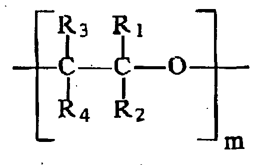 are consisting of patterns 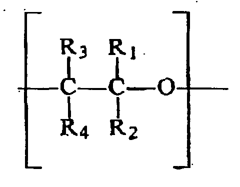 that differ from each another by the nature of R1, R2, R3 and/or R4.

3. Use according to any of claims 1 or 2 **characterized in that**, in formula I, hydrocarbon radicals R1, R2, R3, R4 are alkyl radicals with 1 to 30 carbon atoms.

4. Use according to any of claims 1 to 3 **characterized in that** in formula I n is a number from o to 10 and m is a number from 1 to 30.

5. Use as detergent additive in a gasoline of a composition consisting of a mixture of several compounds as defined in any of claims 1 to 4 which differ from each another by the value of n and/or the nature of at least one radical R1 to R4.

## Patentansprüche

1. Verwendung als Detergenszusatz in einem Benzin von einer Verbindung, die der folgenden allgemeinen Formel (I) entspricht: 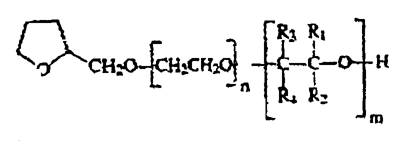 in welcher n eine Zahl von 0 bis 20, R1, R2, R3 und R4 ein Wasserstoffatom oder ein Kohlenwasserstoffrest darstellen, z.B. Alkyl mit 1 bis 30 Kohlenstoffatomen, wobei wenigstens einer der R1, R2, R3 und R4 ein Kohlenwasserstoffrest ist und m eine Zahl von 1 bis 30 ist.

2. Verwendung nach Anspruch 1, **dadurch gekennzeichnet, dass** in der allgemeinen Formel (I) die Kettenbildungen 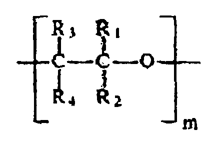 aus Motiven 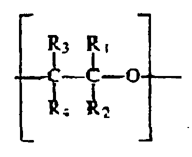 bestehen, die sich durch die Natur von R1, R2, R3 und/oder R4 voneinander unterscheiden.

3. Verwendung nach Anspruch 1 oder 2, **dadurch gekennzeichnet, dass** in der Formel (I) die Kohlenwasserstoffreste R1, R2, R3 und R4 Alkylreste mit 1 bis 30 Kohlenstoffatomen sind.

4. Verwendung nach einem der Ansprüche 1 bis 3, **dadurch gekennzeichnet, dass** in der Formel (I) n eine Zahl von 0 bis 10 ist und m eine Zahl von 0 bis 30 ist.

5. Verwendung als Detergenszusatz in einem Benzin von einer Zusammensetzung, die aus einem Gemisch mehrerer Verbindungen besteht, wie sie in einem der Ansprüche 1 bis 4 definiert sind, die sich voneinander durch den Wert von n und/oder durch die Natur von wenigstens einem der Reste R1 bis R4 unterscheiden.
